# FASCICULE DE BREVET EUROPEEN

(11) **EP 1 189 547 B1**
(45) Date de publication et mention de la délivrance du brevet: **14.01.2009**
(21) Numéro de dépôt: 00929192.3
(22) Date de dépôt: 02.06.2000
(51) Int. Cl.: A61C 1/08, A61C 3/02, A61C 8/00

(54) **DISPOSITIF DE SECURITE COMPORTANT UNE BUTEE POUR OUTIL DE FORAGE UTILISABLE NOTAMMENT EN CHIRURGIE DENTAIRE ET DISPOSITIF DE PRECALIBRAGE ET DE MEMORISATION DE LA PROFONDEUR DE FORAGE**
VORRICHTUNG ZUM BEGRENZEN DES EINDRINGENS EINES BOHWERKZEUGS IN DER ZAHNCHIRURGIE, SOWIE VORRICHTUNG ZUM KALIBRIEREN UND SPEICHERN DER EINDRINGTIEFE
SECURITY DEVICE COMPRISING A STOP MEMBER FOR DRILLING INSTRUMENT USED IN PARTICULAR IN DENTAL SURGERY AND DEVICE PRE-CALIBRATING AND STORING DRILLING DEPTH

(30) Priorité: 03.06.1999 CH 104399
(43) Date de publication de la demande: 27.03.2002
(73) Titulaire: Arsline S.A., 6833 Vacallo (CH)
(72) Inventeur: TURRI, Achille, CH-6834 Morbio Inferiore (CH)
(74) Mandataire: AMMANN PATENTANWÄLTE AG BERN
(86) Numéro de dépôt international: PCT/CH2000/000309
(87) Numéro de publication internationale: WO 2000/074585

(56) Documents cités:
- DE-C- 567 385
- US-A- 4 526 542

## Description

La présente invention porte sur un dispositif de sécurité comportant une butée avec un outil de forage utilisable notamment en chirurgie dentaire selon le préambule de la revendication indépendante 1 et comme divulgué dans US 4 526 542. L'invention concerne également un dispositif de précalibrage et de mémorisation de la profondeur de forage selon le préambule de la revendication 9, qui s'inscrit directement dans le concept d'un processus avantageux d'utilisation dudit dispositif de sécurité.

Un domaine d'application privilégié de ces dispositifs concerne l'implantologie dentaire.

On sait que lors de certaines interventions - notamment le traitement de canaux radiculaires, de certaines lésions dentaires, de tissus durs dentaires ou encore de maxillaires, (par exemple en vue de la mise en place d'un implant dentaire) - la pénétration, le fraisage ou le perçage, pratiqués au moyen d'un outil spécifique (instrument d'endodontie, fraise ou foret dentaire, foret pour l'implantologie, etc.), doivent être effectués sur une profondeur bien définie, que détermine le praticien en fonction des spécificités que présente un patient.

La profondeur définie précitée doit être effectivement atteinte, mais aussi ne pas être dépassée. Cette condition revêt une importance toute particulière dans le domaine de l'implantologie dentaire, car il est impérieux, là, que soit assurée la fiabilité de la tenue de l'infrastructure destinée à être enfoncée dans le maxillaire pour supporter une prothèse dentaire fixe, d'une part, et que soit écarté tout risque de lésion de structures avoisinantes, d'autre part.

Pour ces raisons, les fabricants proposent différents dispositifs visant à répondre à ces contraintes.

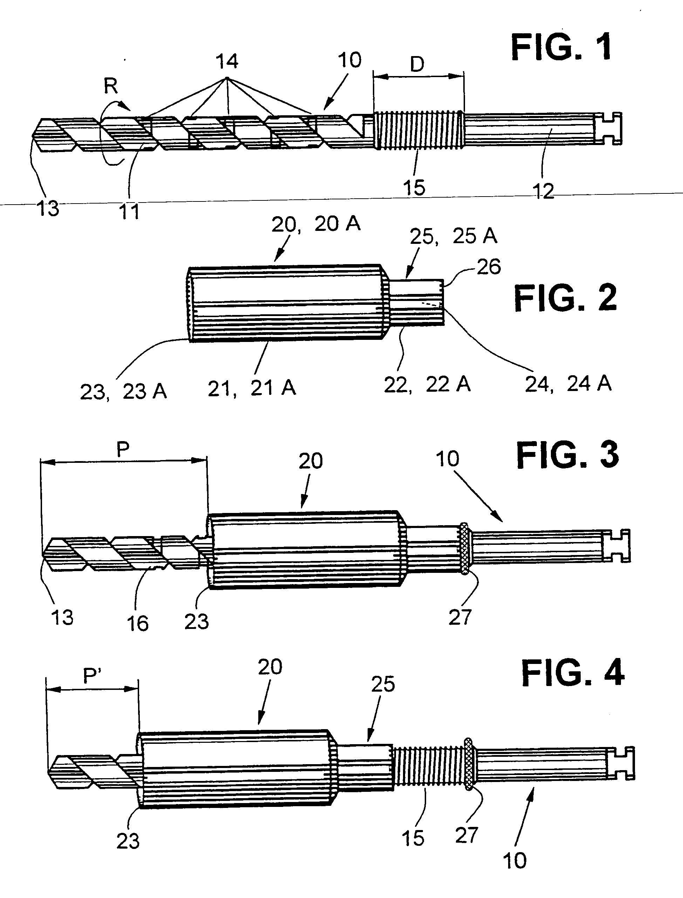

Ainsi, selon un dispositif très simple, mais aussi très fréquemment utilisé en implantologie dentaire, l'outil comporte des marquages en forme de rainures, colorées ou non, pratiquées à différentes distances P₁, P₂, ..., Pₙ, sur la partie active, c'est-à-dire coupante du foret, de sorte qu'un terme puisse être mis à l'opération de forage au bon moment, le praticien voyant que la profondeur qu'il a préalablement déterminée est atteinte (marquages visuels portant la référence 14 dans la figure 1 jointe).

Selon d'autres dispositifs, plus sophistiqués (que l'on va brièvement commenter ci-après), ces outils sont pourvus de butées, observation devant toutefois être faite qu'aucun de ces dispositifs de l'art antérieur n'est adapté à l'implantologie dentaire, ni d'ailleurs prévu pour être mis en oeuvre dans ce domaine spécial.

EP-O 515 274 décrit un outil, en l'occurrence un foret hélicoïdal utilisé en chirurgie dentaire, dont la partie coupante (7) comporte des rainures transversales pouvant remplir la fonction de repère optique de la profondeur de forage. Le praticien peut placer un anneau de butée (8, 9) sur la rainure adéquate, c'est-à-dire sur celle répondant à la profondeur du pertuis qu'il prévoit de pratiquer, ce qui lui permettra d'exercer un meilleur contrôle du forage de ladite profondeur, grâce au positionnement de la butée, laquelle reste ainsi fixée pour l'opération à effectuer.

L'enseignement livré par FR-2 613 212 est analogue, à la différence que la partie non travaillante (1b) comporte une pluralité d'encoches périphériques et transversales, dans lesquelles peut être positionné un clip, ce dernier constituant une butée fixe de profondeur permettant d'abord de déterminer, puis de contrôler la longueur de la partie de l'instrument devant pénétrer dans la dent.

Selon une variante divulguée dans US-4,526,542, la butée est vissée sur la tige de la fraise, jusqu'à ce qu'elle soit bloquée contre la face arrière de la partie coupante, l'orientation du filetage/taraudage étant inverse de celle de la rotation de la fraise, afin de conférer à la butée la fonction de contre-écrou. Au contact d'une surface avoisinante, la butée aura tendance à se bloquer davantage sur l'épaulement que présente la partie travaillante à sa partie supérieure, ce qui, on le verra plus loin, va à l'encontre du but recherché par l'inventeur de la présente invention.

La demande internationale WO 98/03125 montre un foret dont la partie travaillante comporte des dépressions calibrées espacées (par rapport à l'axe du foret) d'un millimètre chaque fois. Une butée en forme d'anneau peut être déplacée le long de ladite partie et peut s'immobiliser à hauteur de chaque dépression, une cheville agencée dans la butée pouvant s'y enclipser sous l'action d'un ressort.

Ces dispositifs permettant d'afficher la profondeur de forage ou de fraisage que le praticien prévoit de faire, c'est-à-dire de positionner la butée de l'instrument en fonction de cette profondeur, constituent dans le même temps un élément de sécurité puisqu'il s'agit, une fois l'instrument ajusté, d'assurer que ladite profondeur ne soit pas dépassée lors de l'opération.

Mais tous ces dispositifs à butée connus présentent, pour le spécialiste en implantologie dentaire qui souhaite effectuer des forages en vue de la pose d'implants, au moins trois sortes d'inconvénients. D'abord, dans la mesure où une butée est tout simplement fixée à une position prédéterminée de travail, les dispositifs de l'art antérieur, outre le fait que la précision d'affichage de la profondeur n'est pas satisfaisante, ne permettent pas toujours une exploitation optimale de la profondeur du site opératoire. Ensuite, ces dispositifs risquent, dans les cas où les conditions de travail ne sont pas optimales, de provoquer l'ovalisation du pertuis, donc de compromettre la stabilité primaire de l'implant, un résultat satisfaisant notamment quant à la tenue de l'implant n'étant alors plus assuré (voir infra). Enfin, ils sont inintéressants sur la plan économique.

Même si ces trois groupes d' inconvénients se conçoivent aisément à l'examen des antériorités, il n'est pas inutile de les expliciter plus avant:

Lorsque l'outil - par définition soumis à une force d'application au cours de sa mise en oeuvre - atteint la profondeur de forage définie, la butée - si elle rencontre une surface inclinée par rapport à l'axe de l'outil, ou une surface de forme irrégulière, par endroits concave ou convexe (ce qui, en implantologie, est très souvent le cas, s'agissant de poser des implants dentaires sur un sommet de crête osseuse de diamètre à peine plus large que celui de l'implant) - risque de glisser sur ladite surface. La probabilité de survenance de ce risque est tout autre que négligeable, étant observé qu'elle est d'autant plus grande dans les cas où l'os soumis au forage est de type très spongieux et lâche ainsi que dans les cas, très fréquents, où la partie plus haute du sommet de la crête osseuse (correspondant toujours à son côté lingual) est constituée d'os cortical, dur, tandis que la partie basse (son coté vestibulaire, dit aussi buccal) est plus tendre (os peu corticalisé). Ce risque, s'il survient, se traduira inéluctablement par un dérapage, c'est-à-dire une dérive latérale de l'axe de forage, ce qui provoquera une ovalisation du pertuis.

Ce grave inconvénient que comportent les dispositifs connus sera une cause de défaut de stabilité primaire de l'implant.

Les techniques et connaissances en implantologie sont en train d'évoluer. D'une part, vers l'utilisation de techniques atraumatiques, lorsqu'il s'agit de la préparation de pertuis osseux aptes à recevoir des "implants-vis en forme de racine". D'autre part, vers l'adaptation de la technique de forage en fonction des quatre densités osseuses (type D1, comparable au bois de chêne, jusqu'au type D4, comparable au polystyrol expansé) rencontrées couramment en implantologie dentaire. Par conséquent, le dentiste, plus précisement l'implantologue, devra disposer d'un très grand nombre de diamètres progressifs de forets (en général de 1.5 à 5 mm). Cela, d'abord, pour éviter des pathologies provoquées par l'échauffement de l'os dense engendré par un alésage trop rapide du pertuis osseux. Ensuite, pour adapter les préparations aux différents diamètres d'implants actuellement à disposition. Enfin, lorsqu'on est en présence d'os spongieux et lâche, pour produire des pertuis osseux suffisamment étroits de manière à permettre l'insertion d'un implant qui puisse avoir une bonne stabilité primaire tout en ne risquant pas la fracture de la trabéculation osseuse lors de l'insertion de l'implant, suite à un alésage insuffisant du pertuis osseux.

Or, aucun des dispositifs de sécurité connus limitant la profondeur du forage ne répond à ces considérations.

On observe enfin qu'aucun d'entr'eux ne divulgue de moyens permettant de précalibrer la profondeur de forage prévue et de mémoriser cette profondeur.

Le but de la présente invention est de pallier les inconvénients indiqués et de tenir compte des considérations précitées.

Il est atteint grâce aux moyens définis dans la revendication indépendante 1, tandis que les moyens faisant l'objet des revendications dépendantes permettent une réalisation préférée de l'invention, de surcroît à bon compte. La revendication 9 permet une exploitation optimale du dispositif de sécurité notamment quant à la gestion du temps et au déroulement des opérations lors d'une séance de traitement en implantologie dentaire.

La butée ainsi revendiquée, outre le fait qu'elle est mobile, peut être qualifiée d'"active" par opposition au caractère "passif" des butées connues de l'art antérieur en ce sens que, de la coopération telle que définie entre la butée et l'outil ou instrument de forage, résulte obligatoirement et automatiquement le retrait dudit instrument du pertuis venant d'être réalisé par le praticien, dès que la profondeur voulue est atteinte. Un dépassement de la profondeur de forage préréglée par le positionnement de la butée mobile selon l'invention et tous autres risques (ovalisation, lésions, etc.) sont donc radicalement exclus.

De façon générale, dès que la butée rencontre un obstacle - ce qui entraîne l'arrêt de la rotation de la butée mobile - cette dernière se déplace exclusivement dans le sens d'un raccourcissement de la longueur de la partie travaillante exposée de l'outil, ce qui écarte tout accident.

De même, lors de la mise en rotation de l'outil, si la force d'inertie de la butée arrivait à la faire tourner sur le moyen de coopération (système vis-écrou, selon un exemple) reliant ces deux éléments (outil (en particulier fraise, foret) et butée), le mouvement de rotation relatif qui serait ainsi imprimé à la butée entraînerait un déplacement axial de cette dernière, cela dans un tel sens que ledit déplacement se traduira en un raccourcissement de la portion de la partie travaillante de l'outil qui émergeait initialement hors de la butée, ce qui constitue, notamment en implantologie dentaire, un facteur de sécurité.

Grâce au retrait automatique du foret en fin de course, tout risque de dérive de l'outil - donc d'ovalisation du pertuis due au dérapage de la butée - est écarté. Cet aspect, comme on l'a vu plus haut, est d'une importance fondamentale en implantologie dentaire, surtout en présence d'os peu corticalisé.

Lors de la préparation de pertuis dans l'os très spongieux (que l'on trouve en général dans la partie postérieure des maxillaires, surtout du maxillaire supérieur) la rétraction du foret empêche aussi tout suralésage. Or, il est impératif que l'os tendre ne soit aucunement suralésé. En effet la perte de précision qui est la conséquence d'un suralésage empêche toute stabilité primaire de l'implant, stabilité qui est la condition essentielle pour l'ostéointégration (la création de ce lien biologique stable entre os et implant permettant une mise en charge cliniquement asymptomatique, fonctionnelle et durable de l'implant: il s'agit là d'un principe fondamental et du but recherché en implantologie moderne).

Le réglage de la profondeur de forage avec les dispositifs de butée connus de l'art antérieur, qui se fait par paliers ou à crans, est assez rudimentaire et peu satisfaisant sur le plan de la précision. A l'opposé, avec le dispositif selon l'invention, la liaison de la butée sur la partie non travaillante (tige) de l'instrument, de préférence au moyen d'un microfiletage, permet un réglage micrométrique aisé, fin et précis de la profondeur de forage.

Le réglage micrométrique, réalisé par un précalibrage de la profondeur de forage grâce à un film radiographique ou tomographique à l'échelle 1:1 procure l'avantage d'une exploitation optimale de la profondeur d'os à disposition, sans risque de lésion des structures avoisinantes. Il permet ainsi une correction intraopératoire aisée de la profondeur de forage choisie, par un déplacement micrométrique très exact de la butée, le foret étant en place dans le pertuis osseux, sous contrôle radiographique. Dans l'hypothèse de radiographie digitalisée, ce contrôle et l'éventuelle correction de la profondeur (et donc du repositionnement de la butée), qui s'en suivront pourront être immédiats, ce qui offrira l'avantage supplémentaire d'une meilleure exploitation du temps. Le microfiletage permet enfin de corriger et compenser exactement l'erreur provoquée par la précontrainte de l'arête de la butée avec la partie la plus proche de la surface osseuse (la position d'une butée se prérègle par rapport à l'axe du foret (manoeuvre impossible avec une butée à paliers ou à crans)).

Cet agencement présente également un avantage économique, puisque le diamètre standard des tiges (pour différents diamètres de la partie travaillante) des forets utilisés en implantologie est toujours de 2.2 millimètres, ce qui permet qu'une butée standard soit utilisable pour tout un panel de forets. On complétera le cas échéant, pour d'autres applications, la butée standard par des butées complémentaires adaptées à d'autres diamètres de tiges. Dans tous les cas, le dispositif selon l'invention offre la possibilité de pouvoir se limiter à un nombre très limité de butées, voire à un seul type standard, pour couvrir pratiquement tous les diamètres de forets (particularité qui, dans les domaines où l'usure est particulièrement importante, ainsi en implantologie, rend ces dispositifs d'autant plus intéressants).

On va maintenant décrire en détail, à titre d'exemple non limitatif, une forme d'exécution du dispositif selon l'invention, à l'appui du dessin annexé dans lequel
la figure 1 représente un foret sur la tige duquel est rapporté un filetage,
la figure 2 montre une butée mobile selon une forme d'exécution,
les figures 3 et 4 montrent le dispositif de sécurité assemblé, la butée se trouvant dans deux positions différentes,
la figure 5 montre, à plus grande échelle, une butée mobile selon une autre forme d'exécution,
la figure 6A est un foret conventionnel,
la figure 6B montre, à échelle agrandie, un élément intermédiaire de liaison et de coopération entre le foret et la butée, selon une variante d'exécution,
la figure 6C est une coupe selon la ligne VI C - VI C de la figure 6B,
la figure 7 montre le dispositif en phase de travail (fin de phase, la butée arrivant contre une paroi représentée schématiquement), et
les figures 8, 9 et 10 représentent le dispositif de précalibrage et de mémorisation de la profondeur de forage.

Un outil 10, en l'occurrence un foret utilisé plus particulièrement en chirurgie dentaire (voir figure 1), présente, d'une part, une partie active ou coupante 11 (c'est-à-dire la partie travaillante) pourvue sur une longueur d'arêtes tranchantes hélicoïdales usuelles (orientées à droite, non référencées sur la figure) et de marques ou rainures 14 également usuelles sur ce type de foret, et, d'autre part, une tige 12 destinée à être engagée dans un mandrin (ou pièce à main) non représenté(e) permettant de soumettre le foret à un entraînement en rotation. Usuellement, cette rotation est orientée à droite, en regardant dans la direction {tige - partie active} (voir flèche R sur la figure 1), le sens des arêtes de la partie active présentant également cette même orientation. Dans sa partie avant, la tige 12 comporte un filetage sur une distance D définie de manière à permettre l'affichage, au moyen de la butée 20 (voir infra) de toutes les profondeurs de forage usuellement effectuées. Le sens du filetage 15 est inverse du sens de rotation R. Selon l'exemple représenté, le filetage est donc à gauche. Il est avantageux de choisir un microfiletage ou au moins un filetage très fin afin de pouvoir procéder, comme on le verra plus loin, à un réglage micrométrique de la profondeur de forage voulue.

La pièce 20 représentée à la figure 2 est une butée mobile comportant à sa partie arrière 22 un taraudage 24 correspondant au filetage 15 du foret 10, les éléments 22, 24 formant l'équivalent d'un écrou de référence générale 25. L'écrou 25 se prolonge vers l'avant d'un corps tubulaire 21 recouvrant partiellement la partie travaillante 11. La butée 20 présente ainsi, selon cet exemple d'exécution, une forme générale de cloche. Le diamètre intérieur du corps 21 est déterminé de telle manière que la butée puisse être montée sur des forets de différents diamètres (dont les plus fréquemment utilisés varient entre 1.5 mm et 5 mm environ). On peut ainsi concevoir une butée standard, ou éventuellement deux butées standards, l'une permettant de coiffer des forets dont le diamètre de la partie travaillante s'étend entre 1.5 mm et 3,5 mm, l'autre des forets dont le diamètre de la partie travaillante s'étend entre 2.5 mm et 5 mm. Ces deux standards permettront ainsi une application universelle du dispositif, le diamètre de la tige de la plus grande partie des forets utilisés en implantologie étant par ailleurs uniformément égal 2.2 mm.

La butée 20 coopère avec le foret 10, celle-là étant agencée sur celui-ci, à l'instar d'un écrou sur une vis (système vis-écrou 15, 25 impliquant la transformation ou l'accompagnement d'un mouvement en rotation en mouvement en translation et vice-versa, en ce sens notamment que lorsque l'écrou tournant est immobilisé, donc la butée 20, la vis, c'est-à-dire le foret 10 continuant de tourner, se déplace longitudinalement). La butée 20 peut être ajustée finement par le praticien, de sorte que la longueur exposée 16 de la partie travaillante 11, entre la face frontale 23 de la butée et la partie extrême 13 de la partie travaillante du foret 10 corresponde très exactement à une profondeur P souhaitée pour le forage projeté (figure 3).

Le moyen de coopération entre le foret 10 et la butée 20, c'est-à-dire, selon l'exemple, le système vis-écrou 15, 25, doit être déterminé de telle sorte que les forces de frottement qu'il engendre soient juste suffisamment grandes pour que le foret 10, lorsqu'il est entraîné en rotation, entraîne avec lui la butée 20, sans que cette dernière n'amorce un mouvement linéaire (qui serait conséquent à une différence entre la vitesse angulaire de la butée et celle du foret (mouvements relatifs)). Le choix évoqué plus haut quant à la nature et au genre du filet à préférer pour ledit système (filetage micrométrique), outre la précision de positionnement de la butée qu'il permet, sert ou du moins contribue, dans le même temps, à satisfaire cette condition, les forces de frottement devant être telles que le mouvement de retrait du foret s'amorce dès que la butée s'immobilise, parce qu'elle entre en contact avec les tissus avoisinants. Selon les cas, il est possible de pourvoir l'un et/ou l'autre élément du moyen de coopération précité, en l'occurrence le taraudage 24 et/ou le filetage 15, d'un moyen de freinage qui amplifiera les forces de frottement. Une variante consisterait à monter contre la face arrière 26 de l'écrou 25, et sur le filetage 15, une bague ou un anneau torique 27 (figures 3 et 4). Bien entendu tous autres moyens connus de l'homme du métier sont envisageables. A titre d'exemple, selon une autre variante, l'élément 15 ou 25 peut être magnétisé ou être pourvu d'une pastille magnétique, de sorte à obtenir une force de frottement prédéterminée entre lesdits éléments.

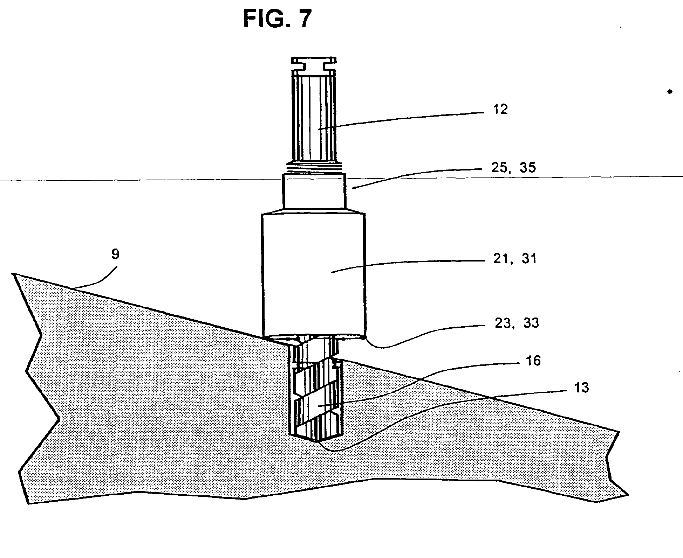

Lors de l'intervention, lorsque la profondeur de forage prévue est atteinte, la face frontale 23 de la butée 20 vient contre la surface avoisinante 9 (voir figure 7). La force d'appui en résultant sur la butée 20, dès qu'elle égalera les forces de frottement de la liaison fraise 10 - butée 20, aura pour effet d'immobiliser la butée 20, ce qui engendrera la rétraction du foret dans le corps tubulaire 21 (voir figure 4, où la portion 16 (égale à P) de la partie travaillante 11 (voir figure 3) est passée à une valeur P' (figure 4), avec P' < P, par le jeu de ladite immobilisation et de la rotation du foret).

Cette réduction sera également signalée par les repères 14 du foret, si ce dernier en est pourvu (voir supra, partie introductive).

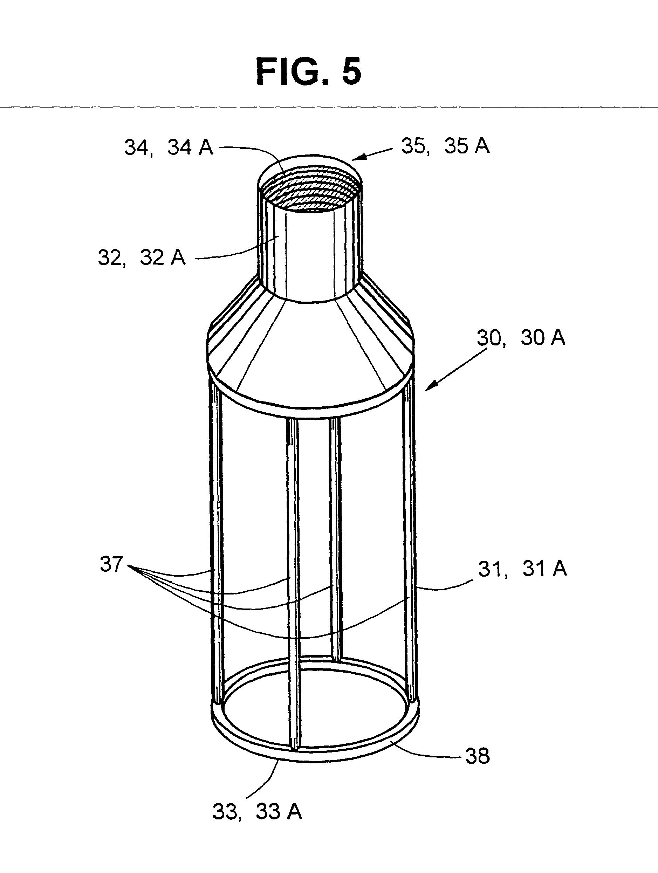

Forme et structure de la butée peuvent évidemment être diverses. A titre d'exemple, la figure 5 montre une butée 30 présentant une structure de basket. On aperçoit la partie arrière taraudée 32, 34, formant un écrou 35, cet élément étant prolongé d'une cage cylindrique 31 comportant un cerclage inférieur 38 (de face frontale 33) relié à l'écrou 35 par quatre barrettes 37. Cette structure, en ne couvrant pas entièrement la partie travaillante 11 du foret 10, en laissant donc cette partie apparente, assure un meilleur refroidissement du foret, puisqu'une surface plus grande de ladite partie travaillante sera alors en contact avec le liquide refroidissement utilisé. Pour le reste, cette butée remplit évidemment les mêmes fonctions que la butée 20: l'écrou 35 coopère avec la vis 15 du foret 10, ce dernier pouvant se retirer dans la cage 31 dès que la partie frontale 33 vient contre la surface avoisinante (portant la référence 9 sur la figure 7).

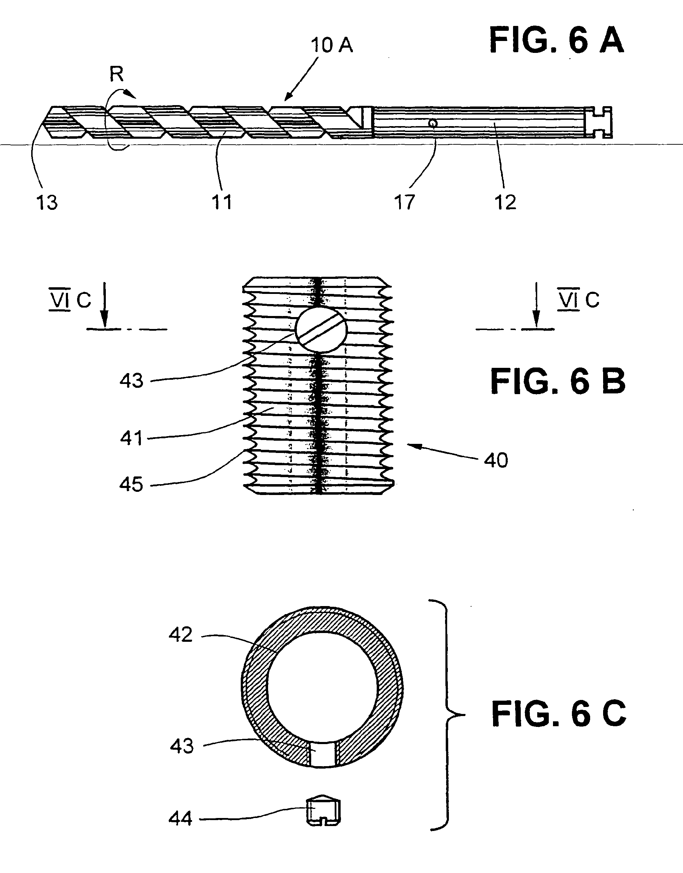

Selon une variante d'exécution (consistant à relier le foret et la butée de manière indirecte), on utilise, d'une part, un foret 10 A (voir figure 6 A), identique en tous points au foret 10 des figures 1 à 4, sauf qu'il ne comporte pas de filetage 15 (ou tout autre moyen permettant de transformer une rotation en déplacement axial) sur la partie 12, mais uniquement, à un endroit défini de cette partie, une creusure 17. D'autre part, on prévoit une pièce intermédiaire amovible, de référence générale 40 (voir figures 6B et 6C, cette dernière étant une coupe selon la ligne VI C de la figure 6 B). Cette pièce 40 présente la forme générale d'une douille 41 de longueur déterminée équivalente à la longueur D (cf. figure 1), tandis que le diamètre de l'alésage intérieur 42 correspond au diamètre de la tige 12 (par définition non filetée selon cette variante d'exécution). La douille 40 comporte un filetage à gauche 45. Elle est destinée à être agencée avec précision sur la tige 12 à l'endroit voulu (qui correspondra avantageusement à celui du filetage 15 du foret 10) et à être bloquée sur celle-ci au moyen d'une micro-vis d'arrêt 44 (figure 6 C) introduite dans le perçage taraudé 43, l'extrémité (non référencée) de la micro-vis d'arrêt pouvant s'engager dans la creusure 17 que comporte la tige 12 (figure 6A) (une telle creusure pourrait d'ailleurs être pratiquée facilement par le praticien même, sur place, au cas où la tige de foret n'en comporterait pas). On conçoit que le caractère amovible de la pièce 40 confère au dispositif une flexibilité et une polyvalence accrues et inégalées. En particulier, il est possible de prévoir des familles de douilles, toutes les douilles présentant le même diamètre extérieur, et le même filetage, mais en revanche des diamètres intérieurs différents (en fonction des diamètres des tiges de forets). De la sorte, une seule butée 20 A, 30 A - de corps 21 A, 31 A et de face frontale 23 A, 33 A (cf. figures 2 et 5), l'écrou 25 A, 35 A, étant formé par la partie arrière 22A; 32 A dont le taraudage 24 A; 34 A correspondra au filetage 45 - pourra être utilisée pour tout un panel de forets.

Bien entendu, de façon générale, la coopération directe ou indirecte butée - tige de foret peut être obtenue par tous autres moyens connus équivalents à ceux décrits à titre d'exemple, c'est-à-dire ayant la fonction de transformer un mouvement de translation en un mouvement de rotation et vice-versa [rainure(s) hélicoïdale(s) avec bille(s) par exemple].

Outre la précision de travail que permet le dispositif selon l'invention, tout risque de dépassement de la profondeur préalablement déterminée, et par conséquent tout risque d'ovalisation du pertuis et de lésion des tissus, son radicalement exclus.

Ces caractères de flexibilité et de polyvalence confèrent en outre au dispositif de sécurité selon l'invention, sur le plan des coûts, un avantage considérable en comparaison avec les dispositifs de sécurité à butées fixes connus. En effet, comme on l'a dit plus haut, en considérant par exemple le domaine de l'implantologie dentaire, où le diamètre des tiges de forets est uniformément égal 2.2 mm, il suffira au praticien spécialisé de se pourvoir d'une seule sorte de butée (butée standard). Dans les autres cas, l'utilisateur pourra se contenter d'un nombre très limité de butées différentes, selon le domaine d'application. Dans tous les cas, il n'y aura pas lieu d'avoir en stock des fraises à butées fixes correspondant à différentes profondeurs.

Par ailleurs, le corps 21; 21 A, 31; 31 A de la butée 20; 20 A; 31; 31 A peut être fabriqué en tout matériau stérilisable, opaque ou transparent.

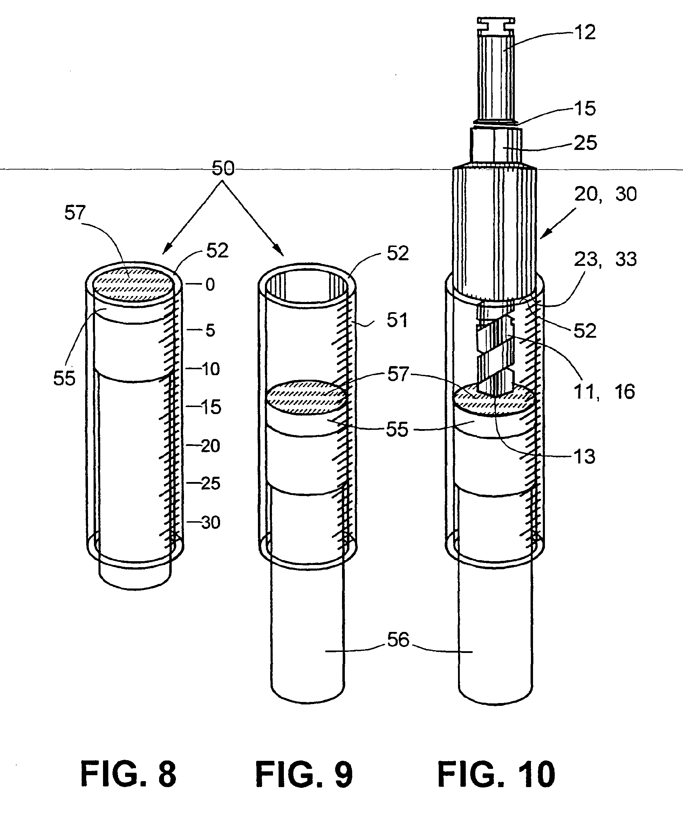

On a vu que le choix du filetage micrométrique permet un réglage très fin de la profondeur de forage définie. D'une part, la distance, c'est-à-dire la mesure de cette profondeur, entre la pointe extrême du foret et la face frontale de la butée peut être effectuée de diverses manières. Mais d'autre part, il peut être avantageux de précalibrer et de mémoriser lors d'une intervention les données des profondeurs de forage de plusieurs implants. A cette fin, on prévoit une série de jauges spécialement conçues pouvant être disposées dans un bloc ou box adéquat et utilisables selon le procédé qui va être décrit. Chaque jauge 50 (voir figures 8, 9 et 10) comprend un cylindre 51 gradué en millimètres (de zéro à trente mm par exemple), en matière transparente, stérilisable en autoclave ou en chemiclave, dans lequel est agencé un piston 55 (dont la face supérieure porte la référence 57) muni de préférence d'une tige de piston 56 permettant une remise à zéro aisée. La graduation 0 mm est à fleur avec la face supérieure 52 du cylindre 51. Le diamètre extérieur du cylindre 51 est approximativement égal au diamètre extérieur de la butée 20; 30, de sorte que la face frontale 23, 33 de la butée 20, 30 puisse venir juste contre la partie supérieure 52 de la jauge (cf. figure 10). Dans un premier temps, la face supérieure 57 du piston 55 se trouve en regard de la marque zéro (figure 8). Dans un deuxième temps, le praticien place l'extrémité 13 de la partie travaillante 11 du foret contre le piston 55, chassant ainsi ce dernier dans le corps du cylindre jusqu'à ce que la surface 57 du piston soit en regard de la marque correspondant à la profondeur de forage prévue (par exemple 15 mm, voir figure 10). Puis il agit sur la butée en la tournant jusqu'à ce que la face frontale 23, 33 de celle-ci arrive contre le bord 52 du cylindre: la butée est en place en vue l'opération de forage projetée relative à la profondeur affichée. Selon les cas, c'est-à-dire, lorsque le practicien prévoit de faire une série de forages, il apparaît avantageux de précalibrer et de mémoriser le choix des différentes profondeurs. A cette fin, le praticien utilisera autant de jauges que de profondeurs prévues, concept particulièrement intéressant lors d'implantations dentaires multiples chez un même patient et au cours d'une même séance de traitement.

## Revendications

1. Dispositif de sécurité (20, 30) avec un outil de forage rotatif (10) utilisable notamment en chirurgie dentaire et comportant une butée, cette butée étant reliée audit outil par des moyens de liaison (15, 25) et présentant une position définie en fonction d'une profondeur voulue de forage, de sorte à pouvoir empêcher un dépassement de cette profondeur lors de la mise en oeuvre de l'outil pourvu de ce dispositif, **caractérisé en ce que** la liaison est assurée directement ou indirectement par des moyens permettant une transformation de mouvements, de sorte que l'outil de forage (10, 10 A) animé d'un mouvement de rotation se retire vers l'arrière par rapport à la direction de forage dès que la butée (20; 30; 20 A; 30 A) est immobilisée par l'entrée en contact de la partie frontale (23; 33; 23 A; 33 A) de celle-ci avec une surface avoisinante (9).

2. Dispositif de sécurité selon la revendication 1, **caractérisé en ce que** les moyens de transformation de mouvements constituent un système vis-écrou (15, 25), le sens du ou des filets dudit système et le sens de rotation du foret en phase de travail étant inverses.

3. Dispositif de sécurité selon la revendication 2, **caractérisé en ce que** la vis est formée d'un filetage (15) rapporté sur la tige (12) du foret (10) et que la butée (20, 30) comporte une partie arrière (22; 32) pourvue d'un taraudage correspondant (24; 34) pour former l'écrou (25; 35).

4. Dispositif de sécurité selon la revendication 2, **caractérisé en ce que** la vis est formée d'un élément intermédiaire (40) en forme de douille sur le corps (41) de laquelle est rapporté un filetage (45), cet élément étant destiné à être agencée sur la tige (12) du foret (10 A), et que la butée (20 A; 30 A) comporte une partie arrière (22 A; 32 A) pourvue d'un taraudage correspondant (24 A; 34 A) pour former l'écrou (25 A; 35 A).

5. Dispositif de sécurité selon l'une des revendications 1 à 4, **caractérisé en ce que** la butée (20; 30; 20 A; 30 A) présente un corps (21; 31; 21 A; 31 A) pouvant s'étendre au moins partiellement autour de la partie travaillante (11) de l'outil de forage (10, 10 A), la profondeur de forage prévue correspondant à une portion (16) émergeant de la face frontale (23; 33; 23 A; 33 A) de la butée, la profondeur pouvant être préréglée par action sur lesdits moyens de coopération, la longueur de la portion (16) allant diminuant lorsque ladite face frontale rencontre la paroi avoisinante (9).

6. Dispositif de sécurité selon l'une des revendications 1 à 4, **caractérisé en ce que** le corps de butée se présente sous une forme tubulaire pleine (21; 21 A) ou sous une forme de cage ajourée (31; 31 A).

7. Dispositif de sécurité selon la revendication 6,
**caractérisé en ce que** le corps de butée est fabriqué en un matériau strérilisable, opaque ou transparent.

8. Dispositif de sécurité selon la revendication 6,
**caractérisé en ce que** lesdits moyens de coopération comportent un élément de freinage tel qu'un anneau torique (27).

9. Dispositif de sécurité selon l'une des revendications 1 à 8, **caractérisé en ce que** la profondeur de forage voulue est réglée avant mise en oeuvre au moyen d'un dispositif de précalibrage et de mémorisation comprenant une jauge (50) formée d'un corps transparent gradué (51) et d'un piston (55) pouvant être pourvu d'une tige (56), la face supérieure (52) du corps (51) correspondant à la marque zéro de la graduation et le diamètre extérieur du corps (51) correspondant approximativement au diamètre extérieur de la butée.

10. Procédé de précalibrage et de mémorisation au moyen du dispositif de précalibrage et de mémorisation selon la revendication 9, de la profondeur de forage prévue au moyen d'une jauge, **caractérisé en ce que** la face frontale de la butée est appliquée contre la face supérieure du corps de la jauge et que le piston est chassé à l'intérieur du corps par action des moyens de coopération reliant le foret et la butée, cette action provoquant une poussée exercée par l'extrémité de la partie travaillante du foret sur ledit piston, jusqu'à ce que la face supérieure (57) du piston soit en regard de la graduation correspondant à la profondeur de forage voulue.

## Claims

1. Safety device (20, 30) with a rotary drilling tool (10) for use particularly in dental surgery and comprising a stop, the stop being connected to the tool by connecting means (15, 25) and exhibiting a position that is defined in function of a desired drilling depth so as to be able to prevent this depth being exceeded during use of the tool provided with the device, **characterised in that** the connection is ensured directly or indirectly by means that allow a conversion of movements such that the drilling tool (10, 10 A) set into rotation withdraws to the rear relative to the drilling direction as soon as the stop (20; 30; 20 A; 30 A) is immobilised as the front portion (23; 33; 23 A; 33 A) thereof enters into contact with a neighbouring surface (9).

2. Safety device according to claim 1, **characterised in that** the movement conversion means constitute a screw and nut system (15, 25), the direction of the thread or threads of this system and the direction of rotation of the drill in the working phase being inverse.

3. Safety device according to claim 2, **characterised in that** the screw is formed by a thread (15) provided on the shank (12) of the drill (10) and that the stop (20, 30) has a rear portion (22; 32) provided with a corresponding internal thread (24; 34) to form the nut (25; 35).

4. Safety device according to claim 2, **characterised in that** the screw is formed by an intermediate element (40) in the form of a sleeve on the body (41) of which a thread (45) is provided, this element being intended to be arranged on the shank (12) of the drill (10 A), and that the stop (20 A; 30 A) has a rear portion (22 A; 32 A) provided with a corresponding internal thread (24 A; 34 A) to form the nut (25 A; 35 A).

5. Safety device according to one of claims 1 to 4, **characterised in that** the stop (20; 30; 20 A; 30 A) has a body (21; 31; 21 A; 31 A) that may extend at least partly around the working portion (11) of the drilling tool (10, 10 A), the intended drilling depth corresponding to a portion (16) that projects from the front face (23; 33; 23 A; 33 A) of the stop, the depth being preadjustable by acting upon the cooperating means, and the length of the portion (16) decreasing when the front face meets the neighbouring wall (9).

6. Safety device according to one of claims 1 to 4, **characterised in that** the stop body has a solid tubular form (21; 21 A) or the form of an apertured cage (31; 31 A).

7. Safety device according to claim 6, **characterised in that** the stop body is made of a sterilisable opaque or transparent material.

8. Safety device according to claim 6, **characterised in that** the cooperating means comprise a braking element such as an O-ring (27).

9. Safety device according to one of claims 1 to 8, **characterised in that** the intended drilling depth is adjusted before use by means of a precalibrating and memorising device comprising a gauge (50) formed of a graduated transparent body (51) and of a piston (55) that may be provided with a stem (56), the upper face (52) of the body (51) corresponding to the zero mark of the graduation and the external diameter of the body (51) corresponding to the external diameter of the stop approximately.

10. Method for precalibrating and memorising, by means of the precalibrating and memorising device according to claim 9, the intended drilling depth by means of a gauge, **characterised in that** the front face of the stop is pressed against the upper face of the gauge body and the piston is driven into the body by the action of the cooperating means that connect the drill and the stop, this action causing the end of the working portion of the drill to exert a thrust on the piston until the upper face (57) of the piston is at the graduation that corresponds to the intended drilling depth.

## Patentansprüche

1. Sicherheitsvorrichtung (20, 30) mit einem drehenden Bohrwerkzeug (10) zur Verwendung insbesondere in der Zahnchirurgie und mit einem Anschlag, wobei dieser Anschlag über Verbindungsmittel (15, 25) mit dem genannten Werkzeug verbunden ist und eine in Abhängigkeit von einer gewünschten Bohrtiefe definierte Stellung aufweist, um ein Überschreiten dieser Tiefe bei der Anwendung des mit dieser Vorrichtung ausgerüsteten Werkzeugs verhindern zu können, **dadurch gekennzeichnet, dass** die Verbindung direkt oder indirekt durch Mittel hergestellt wird, die eine Bewegungsumwandlung gestatten, so dass das in Drehung versetzte Bohrwerkzeug (10, 10 A) sich bezüglich der Bohrrichtung nach hinten zurückzieht, sobald der Anschlag (20; 30; 20 A; 30 A) durch die Berührung seines Vorderteils (23; 33; 23 A; 33 A) mit einer benachbarten Oberfläche (9) angehalten wird.

2. Sicherheitsvorrichtung nach Anspruch 1, **dadurch gekennzeichnet, dass** die Mittel zur Bewegungsumwandlung ein System aus Schraube und Mutter (15, 25) bilden, wobei die Richtung des oder der Gewinde(s) dieses Systems und die Drehrichtung des Bohrers in der Arbeitsphase entgegengesetzt sind.

3. Sicherheitsvorrichtung nach Anspruch 2, **dadurch gekennzeichnet, dass** die Schraube aus einem auf dem Schaft (12) des Bohrers (10) angebrachten Gewinde (15) besteht und der Anschlag (20, 30) einen hinteren Teil (22; 32) aufweist, der ein entsprechendes Innengewinde (24; 34) aufweist, um die Mutter (25; 35) zu bilden.

4. Sicherheitsvorrichtung nach Anspruch 2, **dadurch gekennzeichnet, dass** die Schraube aus einem Zwischenstück (40) in Form einer Hülse besteht, auf deren Körper (41) ein Gewinde (45) angebracht ist, wobei dieses Stück auf dem Schaft (12) des Bohrers (10 A) anbringbar ist, und dass der Anschlag (20 A; 30 A) einen hinteren Teil (22 A; 32 A) aufweist, der ein entsprechendes Innengewinde (24 A; 34 A) aufweist, um die Mutter (25 A; 35 A) zu bilden.

5. Sicherheitsvorrichtung nach einem der Ansprüche 1 bis 4, **dadurch gekennzeichnet, dass** der Anschlag (20; 30; 20 A; 30 A) einen Körper (21; 31; 21 A; 31 A) aufweist, der sich mindestens teilweise um den Arbeitsteil (11) des Bohrwerkzeugs (10, 10 A) erstrecken kann, wobei die vorgesehene Bohrtiefe einem über die Stirnseite (23; 33; 23 A; 33 A) des Anschlags vorstehenden Teil (16) entspricht, wobei die Tiefe durch Einwirkung auf die genannten zusammenarbeitenden Mittel voreinstellbar ist und die Länge des Teils (16) abnimmt, wenn die genannte Stirnseite auf die benachbarte Wand (9) trifft.

6. Sicherheitsvorrichtung nach einem der Ansprüche 1 bis 4, **dadurch gekennzeichnet, dass** der Anschlagkörper die Form eines vollwandigen Rohrs (21; 21 A) oder die Form eines durchbrochenen Käfigs (31; 31 A) aufweist.

7. Sicherheitsvorrichtung nach Anspruch 6, **dadurch gekennzeichnet, dass** der Anschlagkörper aus einem sterilisierbaren, undurchsichtigen oder durchsichtigen Material hergestellt ist.

8. Sicherheitsvorrichtung nach Anspruch 6, **dadurch gekennzeichnet, dass** die genannten zusammenarbeitenden Mittel ein Bremselement wie einen O-Ring (27) beinhalten.

9. Sicherheitsvorrichtung nach einem der Ansprüche 1 bis 8, **dadurch gekennzeichnet, dass** die gewünschte Bohrtiefe vor der Anwendung mittels einer Vorkalibrier- und Speichervorrichtung mit einer Lehre (50) eingestellt wird, die aus einem durchsichtigen Körper (51) mit einer Skala und einem Kolben (55) besteht, der einen Schaft (56) aufweisen kann, wobei die Stirnseite (52) des Körpers (51) der Nullmarkierung der Skala entspricht und der Aussendurchmesser des Körpers (51) ungefähr dem Aussendurchmesser des Anschlags.

10. Verfahren zum Vorkalibrieren und Speichern der vorgesehenen Bohrtiefe mittels der Vorkalibrier- und Speichervorrichtung nach Anspruch 9 mittels einer Lehre, **dadurch gekennzeichnet, dass** die Stirnseite des Anschlags gegen die Oberseite des Körpers der Lehre gedrückt und der Kolben durch die Wirkung der den Bohrer und den Anschlag verbindenden zusammenarbeitenden Mittel in den Körper geschoben wird, wodurch das Ende des Arbeitsteils des Bohrers einen Schub auf den Kolben ausübt, bis die Oberseite (57) des Kolbens bei der der gewünschten Bohrtiefe entsprechenden Markierung steht.
